# EUROPEAN PATENT APPLICATION

(11) **EP 4 375 309 A1**
(43) Date of publication of application: **29.05.2024**
(21) Application number: 22845836.0
(22) Date of filing: 14.07.2022
(51) Int. Cl.: C08G 59/22, C08K 5/13, C08L 71/12

(54) **PHENOL MIXTURE, EPOXY RESIN, EPOXY RESIN COMPOSITION, CURED PRODUCT, AND ELECTRICAL/ELECTRONIC COMPONENT**

(30) Priority: 19.07.2021 JP 2021119171
(71) Applicant: Mitsubishi Chemical Corporation, Chiyoda-ku Tokyo 100-8251 (JP)
(72) Inventor: OTA, Kazumasa, Tokyo 100-8251 (JP)
(74) Representative: Hoffmann Eitle
(86) International application number: PCT/JP2022/027636
(87) International publication number: WO 2023/002902

(57) **Abstract**

The present invention addresses the problem of providing: a phenol mixture optimal as a raw material for the production of an epoxy resin that can realize a high heat resistance as required in recent years; and an epoxy resin, an epoxy resin composition, a cured product, and an electrical/electronic component, in which the phenol mixture is used. The problem is solved by: a phenol mixture containing 3,3',5,5'-tetramethyl-4,4'-biphenol as a main component, which phenol mixture further contains more than 0.3% by weight but less than 10.0% by weight of a polyphenylene ether, and 1.3% by weight to 4.0% by weight of tetramethyldiphenoquinone; and an epoxy resin, an epoxy resin composition, a cured product, and an electrical/electronic component, in which the phenol mixture is used.

## Description

### TECHNICAL FIELD

The present invention relates to a phenol mixture. More particularly, the present invention relates to: a phenol mixture used as a raw material for the production of an epoxy resin limited to have a specific formulation for the purpose of providing an epoxy resin that exhibits excellent heat resistance; and an epoxy resin in which the phenol mixture is used, an epoxy resin composition, a cured product, and an electrical/electronic component.

### BACKGROUND ART

Epoxy resins are utilized in a wide range of fields including adhesives, paints, and electrical/electronic materials, since they are cured with various curing agents to yield cured products that are generally excellent in mechanical properties, heat resistance, electrical properties, and the like. Especially, in the field of electrical/electronic materials, tetramethylbiphenol-type epoxy resins are often used for semiconductor sealing applications since they can provide high-value-added sealing materials.

With the recent remarkable technological development in semiconductors, elements such as SiC and GaN have been increasingly used in next-generation power semiconductors; however, since they are used at a higher temperature than the currently mainstream Si elements, an improvement particularly in the heat resistance is demanded so that a sealing material serving as a protective member can withstand a high-temperature environment.

Generally, a tetramethylbiphenol-type epoxy resin can be obtained by a condensation reaction of a biphenol and an epihalohydrin that are raw materials, and 3,3',5,5'-tetramethyl-4,4'-biphenol (hereinafter, may be abbreviated as "TMBPL") is known as a raw material of a tetramethylbiphenol-type epoxy resin.

Patent Documents 1 to 3 disclose 3,3',5,5'-tetramethyl-4,4'-biphenol in which the content of specific impurities is in a specific range, and production methods thereof, and it is described that a tetramethylbiphenol-type epoxy resin was produced by a reaction between TMBPL and epichlorohydrin.

### RELATED ART DOCUMENTS

### PATENT DOCUMENTS

[Patent Document 1] Japanese Unexamined Patent Application Publication No. 2003-327554
[Patent Document 2] Japanese Unexamined Patent Application Publication No. 2004-2830
[Patent Document 3] Japanese Unexamined Patent Application Publication No. S61-268641

### SUMMARY OF THE INVENTION

### PROBLEMS TO BE SOLVED BY THE INVENTION

Cured products obtained from the tetramethylbiphenol-type epoxy resins using TMBPL described in Patent Documents 1 to 3 or epoxy resin compositions thereof have a low glass transition temperature (Tg) and a low 5% weight reduction temperature; therefore, the heat resistance does not tend to be improved.

An object of the present invention is to provide: a phenol mixture optimal as a raw material for the production of an epoxy resin that can realize a high heat resistance as required in recent years; and an epoxy resin, an epoxy resin composition, a cured product, and an electrical/electronic component, in which the phenol mixture is used.

### MEANS FOR SOLVING THE PROBLEMS

The present inventors intensively studied to solve the above-described problems and consequently discovered that, although 3,3',5,5'-tetramethyl-4,4'-biphenol contains various accessory components generated in its production process, and optimization of the reaction conditions, purification, and the like are conventionally performed so as to reduce the accessory components as much as possible, an epoxy resin obtained using 3,3',5,5'-tetramethyl-4,4'-biphenol rather allowed to contain a specific amount of specific accessory components exhibit excellent heat resistance, thereby completing the present invention.

That is, the gist of the present invention resides in the following [1] to [8].
[1] A phenol mixture, containing 3,3',5,5'-tetramethyl-4,4'-biphenol as a main component,
   wherein the phenol mixture further contains more than 0.3% by weight but less than 10.0% by weight of a polyphenylene ether.
[2] The phenol mixture according to [1], further containing 1.3% by weight to 4.0% by weight of tetramethyldiphenoquinone.
[3] An epoxy resin, obtained by reacting the phenol mixture according to [1] or [2] with an epihalohydrin.
[4] An epoxy resin composition, containing 0.01 to 1,000 parts by weight of a curing agent with respect to 100 parts by weight of the epoxy resin according to [3].
[5] The epoxy resin composition according to [4], wherein the curing agent is at least one selected from the group consisting of a phenolic curing agent, an amine-based curing agent, a tertiary amine, an acid anhydride-based curing agent, an amide-based curing agent, and an imidazole.
[6] The epoxy resin composition according to [4] or [5], further containing an epoxy resin that is different from the epoxy resin contained in the epoxy resin composition.
[7] A cured product, obtained by curing the epoxy resin composition according to any one of [4] to [6].
[8] An electrical/electronic component, obtained by curing the epoxy resin composition according to any one of [4] to [6].

### EFFECTS OF THE INVENTION

According to the present invention, the phenol mixture of one embodiment of the present invention and an epoxy resin obtained using the phenol mixture can each yield an epoxy resin cured product having superior heat resistance than a conventional epoxy resin cured product using TMBPL and, therefore, can be used as raw materials optimal for the production of a tetramethylbiphenol-type epoxy resin.

Further, an epoxy resin composition containing the epoxy resin, a cured product obtained by curing the epoxy resin or the epoxy resin composition, and an electrical/electronic component containing the epoxy resin, such as a semiconductor sealing material, are expected to be continuously usable in a temperature range higher than a conventionally used temperature range.

### MODE FOR CARRYING OUT THE INVENTION

Embodiments of the present invention will now be described in detail; however, the following description is merely an example of embodiments of the present invention, and the present invention is not limited thereto within the gist of the present invention. It is noted here that, in the present specification, the expression "to" is used as an expression that includes the numerical or physical property values stated before and after "to".

### [Phenol Mixture]

The phenol mixture according to one embodiment of the present invention is a phenol mixture containing 3,3',5,5'-tetramethyl-4,4'-biphenol as a main component, which phenol mixture further contains more than 0.3% by weight but less than 10.0% by weight of a polyphenylene ether (hereinafter, may be abbreviated as "PPE") and 1.3 to 4.0% by weight of tetramethyldiphenoquinone (hereinafter, may be abbreviated as "DPQ").

The phenol mixture of the present embodiment is called a "phenol mixture" since it contains TMBPL as a main component along with plural accessory components, and the phenol mixture of the present embodiment may encompass a phenol mixture that is obtained as a "mixture" composed of TMBPL and other component(s) by a process not aimed at obtaining a phenol compound. It is noted here that, in the art, even a mixture or the like not consisting of a single component is sometimes simply expressed as, referred to as, or marketed as "phenol" or "phenol compound".

The reasons why an epoxy resin obtained using the phenol mixture of the present embodiment exhibits excellent heat resistance are not clear; however, they are presumed as follows.

That is, it is presumed that the incorporation of a relatively high-molecular-weight PPE into the phenol mixture of the present embodiment at a specific ratio somehow contributes to an increase in the crosslinking density of a cured product that is produced by curing an epoxy resin obtained using the phenol mixture, consequently exerting an effect in improvement of the heat resistance. A greater amount of such a PPE leads to a further increase in the crosslinking density of the cured product, and the Tg and the 5% weight reduction temperature of the cured product thus tend to be increased, whereas a smaller amount of such a PPE leads to a further reduction in the amount of a high-molecular weight component, and the Tg and the 5% weight reduction temperature of the cured product thus tend to be decreased.

Further, when the phenol mixture of the present embodiment contains DPQ, since DPQ itself has a melting point of 222°C and is expected to have a high boiling point, it is thought that, by incorporating DPQ at a specific ratio, a cured product having excellent heat resistance with sufficiently high Tg and 5% weight reduction temperature is obtained in the production of a cured product by curing an epoxy resin obtained using the phenol mixture. A higher content of DPQ leads to a further increase in the crosslinking density, and the Tg and the 5% weight reduction temperature of the resulting cured product thus tend to be increased, whereas a lower content of DPQ tends to further decrease the Tg and the 5% weight reduction temperature of the cured product.

From the above-described reasons, the content of PPE is preferably 0.5% by weight to 8.0% by weight, more preferably 1.0% by weight to 7.5% by weight, still more preferably 1.5% by weight to 5.0% by weight.

The content of DPQ is preferably 1.5% by weight to 3.5% by weight, more preferably 2.0% by weight to 3.0% by weight.

The phenol mixture of the present embodiment contains TMBPL as a main component, and the expression "as a main component" used herein specifically means that the phenol mixture contains TMBPL in an amount of preferably not less than 80% by weight, more preferably not less than 85% by weight, still more preferably not less than 90% by weight.

In addition to TMBPL as the main component, PPE, and DPQ, the phenol mixture of the present embodiment may contain other plural accessory components as well. The accessory components are not particularly limited, and preferred examples thereof include 4-(2,6-dimethylphenoxy)-2,6-dimethylphenol (hereinafter, may be abbreviated as "ED") and 2,6-xylenol (hereinafter, may be abbreviated as "XNL").

When the phenol mixture contains ED, the content thereof is not particularly limited; however, it is preferably 0.2% by weight or less, more preferably 0.1% by weight or less. It is preferred to control the content of ED having a phenolic hydroxyl group that is monofunctional to be 0.2% by weight or less since this gives a cured product having excellent heat resistance with sufficiently high Tg and 5% weight reduction temperature in the production of a cured product of an epoxy resin.

When the phenol mixture contains XNL, the content thereof is not particularly limited; however, it is preferably 0.1 to 3.4% by weight, more preferably 0.3 to 2.5% by weight, still more preferably 0.5 to 2.0% by weight. A lower content of 2,6-xylenol having a phenolic hydroxyl group that is monofunctional gives a cured product having superior heat resistance with sufficiently high Tg and 5% weight reduction temperature in the production of a cured product of an epoxy resin, whereas a higher content of 2,6-xylenol tends to further improve the productivity in the industrial-scale production of the phenol mixture.

The formulation of the phenol mixture can be determined by the method described below in the section of Examples.

A second embodiment of the present invention is a phenol mixture containing 3,3',5,5'-tetramethyl-4,4'-biphenol as a main component, which phenol mixture further contains 1.3 to 4.0% by weight of DPQ and 0.1 to 3.4% by weight of XNL. Preferred ranges of the contents of 3,3',5,5'-tetramethyl-4,4'-biphenol, DPQ, and XNL are the same as in the above-described embodiment.

A third embodiment of the present invention is a phenol mixture containing 3,3',5,5'-tetramethyl-4,4'-biphenol as a main component, which phenol mixture further contains 0.2% by weight or less of ED and 0.1 to 3.4% by weight of XNL. Preferred ranges of the contents of 3,3',5,5'-tetramethyl-4,4'-biphenol, ED, and XNL are the same as in the above-described embodiment.

A fourth embodiment of the present invention is a phenol mixture containing 3,3',5,5'-tetramethyl-4,4'-biphenol as a main component, which phenol mixture further contains 0.2% by weight or less of ED and 1.3 to 4.0% by weight of DPQ. Preferred ranges of the contents of 3,3',5,5'-tetramethyl-4,4'-biphenol, ED, and DPQ are the same as in the above-described embodiment.

### [Method of Producing Phenol Mixture]

A method of producing each phenol mixture of the first to the fourth embodiments is not particularly limited, and examples of a representative production method include a production method that includes the step of oxidatively dimerizing 2,6-xylenol in a surfactant-containing alkaline water solvent in the presence of a metal catalyst and an oxidizing agent.

The amount of water used as a reaction solvent is usually 0.5 to 10 kg, preferably 1 to 5 kg, per 1 kg of 2,6-xylenol. Examples of the surfactant include fatty acid soaps, alkyl sulfonates, alkylbenzene sulfonates, and alkyl sulfates. The surfactant is preferably sodium lauryl sulfate. The amount of the surfactant to be used is usually in a range of 0.01 to 50 mmol, preferably in a range of 0.1 to 10 mmol, per 1 mol of 2,6-xylenol.

Examples of an alkaline substance include: alkali metal hydroxides, such as sodium hydroxide and potassium hydroxide; alkali metal carbonates, such as sodium carbonate; bicarbonates; boron compounds, such as sodium borate and borax; alkali metal phosphates, such as sodium phosphate, sodium hydrogen phosphate, potassium phosphate, and potassium hydrogen phosphate; and amine bases, such as triethylamine and pyridine. Among these, a boron compound is preferred from the standpoint of yield and selectivity, and borax is particularly preferred. The alkaline substance is usually used in such an amount that the pH of the reaction system is maintained in a range of 8 to 11.

As the metal catalyst, a copper compound is preferably used. The copper compound may be monovalent or divalent, and specific examples thereof include copper halide, copper hydroxide, copper sulfate, copper nitrate, copper carboxylate, and copper alkylsulfate. The amount of the metal catalyst to be used is 0.005 to 0.1 mmol, preferably 0.01 to 0.06 mmol, per 1 mol of 2,6-xylenol.

As the oxidizing agent, in addition to oxygen gas, an oxygen-containing gas such as air is used, and oxygen is preferably used. The amount of the oxidizing agent to be used is in a range of 0.01 to 1 mol, preferably 0.1 to 0.6 mol, per 1 mol of 2,6-xylenol. By controlling the amount of the oxidizing agent to be in a specific range, the ratio of 4-(2,6-dimethylphenoxy)-2,6-dimethylphenol, tetramethyldiphenoquinone, and 2,6-xylenol in the resulting phenol mixture can be obtained in a specific range. The reaction temperature is usually 50 to 100°C, and the reaction pressure is in a range of the atmospheric pressure to 30 atm, although this varies depending on the oxygen concentration in a gas phase. The reaction time is usually 1 to 24 hours, preferably 5 to 15 hours.

The production method preferably includes the following steps after the step of oxidatively dimerizing 2,6-xylenol. That is, the resulting reaction solution is heated while maintaining the pH at 7.5 to 9.0 to remove water and unreacted 2,6-xylenol by distillation. In this case, pH adjustment is preferably performed after purging the reaction system with an inert gas such as nitrogen. An acid used for the pH adjustment is not particularly limited; however, a mineral acid is generally used. Particularly, sulfuric acid or hydrochloric acid is preferred. By adjusting the pH to be in the above-described range, the contents of polyphenylene ether and other components can be controlled in the respective prescribed ranges of each embodiment. A reaction pressure is not particularly limited, and the reaction can be carried out under normal pressure or reduced pressure.

Subsequently, the resulting product liquid is adjusted to have a pH of 2 to 6.9 with an addition of an acid thereto, an alcohol is then added, and this is followed by mixing and stirring to obtain a mixed liquid. As the alcohol, a lower alcohol having 1 to 4 carbon atoms, such as methanol, ethanol, n-propyl alcohol, isopropyl alcohol, or n-butanol, is preferably used. In this case, the weight ratio of the alcohol and water (alcohol/water) is usually 1/1 to 10/1, preferably 2/1 to 5/1. In the preparation of the mixed liquid, accompanying water from a reaction slurry is utilized; however, water is added when each phenol mixture is recovered as a solid.

By controlling the mixing ratio of water and the alcohol to be in the above-described range, the yield of each phenol mixture of the first to the fourth embodiments can be maintained high. The concentration of each phenol mixture in the mixed liquid is usually 5 to 50% by weight. The temperature during the mixing and stirring is usually 40 to 100°C, preferably 50 to 90°C. The duration of the mixing and stirring is usually 0.1 to 5 hours, preferably 0.3 to 2 hours.

Thereafter, the thus obtained mixed liquid is subjected to solid-liquid separation to recover each phenol mixture. This solid-liquid separation is performed by means of filtration, centrifugation, or the like. The temperature of the mixed liquid at the time of the solid-liquid separation is usually 35 to 70°C, preferably 40 to 65°C. In addition, each phenol mixture can be recovered by heating and pressure reduction.

### [Epoxy Resin]

The epoxy resin according to another embodiment of the present invention is an epoxy resin obtained by reacting the phenol mixture according to any one of the first to the fourth embodiments of the present invention with an epihalohydrin.

The epoxy resin of the present embodiment is produced from the phenol mixture according to any one of the first to the fourth embodiments of the present invention and, during the reaction with an epihalohydrin, some of PPE, DPQ, and the like contained in the phenol mixture contribute to the reaction, remain as is without contributing to the reaction, or are removed from the system by distillation during the reaction. Therefore, in the resulting epoxy resin, it is difficult to determine the presence and the forms of PPE and DPQ contained in the phenol mixture, or to identify the structure of the epoxy resin by an analysis.

It is noted here that the epoxy resin of the present embodiment may contain a repeating structure or has a single molecular structure and, in the art, an epoxy compound of either type may be expressed and marketed as "epoxy resin". Further, in the art, a mixture that further contains an epoxy resin different from the epoxy resin of the present embodiment may be simply referred to as "epoxy resin".

### [Epoxy Equivalent]

The epoxy resin of the present embodiment preferably has an epoxy equivalent of 186 to 189 g/equivalent (hereinafter, may be denoted as "g/eq") from the standpoint of improving the heat resistance of a cured product obtained from the epoxy resin. It is believed that excellent curing properties are obtained by controlling the epoxy equivalent to be in the above-described specific range.

The epoxy equivalent increases depending on the amounts of an epihalohydrin and an alkali metal hydroxide that are used in the reaction.

In the present invention, the "epoxy equivalent" is defined as "the mass of an epoxy resin containing one equivalent of epoxy groups", and can be measured in accordance with JIS K7236.

### [Method of Producing Epoxy Resin]

A method of producing the epoxy resin of the present embodiment is not particularly limited, and examples thereof include a method of obtaining the epoxy resin by reacting the phenol mixture according to any one of the first to the fourth embodiments of the present invention with an epihalohydrin in the presence of an alkali metal hydroxide.

When the epoxy resin is produced by this method, at least the phenol mixture according to any one of the first to the fourth embodiments of the present invention and an epihalohydrin are used as raw materials, and a polyvalent hydroxy compound other than the above-described phenol mixture (this polyvalent hydroxy compound may be hereinafter referred to as "other polyvalent hydroxy compound") may also be used in combination to produce the epoxy resin. However, from the standpoint of improving the heat resistance of a cured product obtained from the epoxy resin of the present embodiment, it is preferred to use the phenol resin composition of the present embodiment singly.

The term "polyvalent hydroxy compound" used herein is a generic term for a phenol compound having two or more hydroxy groups.

Examples of the other polyvalent hydroxy compound include: various polyhydric phenols (excluding the phenol mixtures according to the first to the fourth embodiments of the present invention), such as bisphenol A, bisphenol F, bisphenol S, bisphenol AD, bisphenol AF, hydroquinone, resorcin, methyl resorcin, biphenol, dihydroxynaphthalene, dihydroxydiphenyl ether, thiodiphenols, phenol novolac resins, cresol novolac resins, phenol aralkyl resins, biphenyl aralkyl resins, naphthol aralkyl resins, terpene phenol resins, dicyclopentadiene phenol resins, bisphenol A novolac resins, naphthol novolac resins, brominated bisphenol A, and brominated phenol novolac resins; and various phenolic resins, such as polyhydric phenolic resins obtained by condensation reaction of a phenol and an aldehyde such as benzaldehyde, hydroxybenzaldehyde, crotonaldehyde, or glyoxal, polyhydric phenolic resins obtained by condensation reaction of a xylene resin and a phenol, and co-condensation resins of a heavy oil or a pitch, a phenol, and a formaldehyde.

There among, preferred examples include phenol novolac resins, phenol aralkyl resins, polyhydric phenolic resins obtained by condensation reaction of a phenol and hydroxybenzaldehyde, biphenyl aralkyl resins, and naphthol aralkyl resins.

The amount of the epihalohydrin to be used is usually 2 to 10.0 equivalents, particularly preferably 4 to 8 equivalents, per equivalent of hydroxy groups of all polyvalent hydroxy compounds that is a total of the above-described phenol mixture used as a raw material and the other polyvalent hydroxy compound(s) used as required. When the amount of the epihalohydrin is equal to or greater than the above-described lower limit, the molecular weight-increasing reaction is easily controllable, so that the resulting epoxy resin can be provided with an appropriate epoxy equivalent, which is preferred. Meanwhile, when the amount of the epihalohydrin is equal to or less than the above-described upper limit, the production efficiency tends to be improved, which is preferred. As the epihalohydrin, epichlorohydrin or epibromohydrin is usually used; however, epichlorohydrin is preferably used in the present embodiment.

The amount of the alkali metal hydroxide to be used is usually 0.8 to 1.6 equivalents, preferably 1.0 to 1.4 equivalents, per equivalent of hydroxy groups of all polyvalent hydroxy compounds used as raw materials of the epoxy resin. The alkali metal hydroxide in an amount corresponding to the above-described amount of use is added and reacted in the form of a solid or an aqueous solution. When the amount of the alkali metal hydroxide is equal to or greater than the above-described lower limit, unreacted hydroxy groups and the resulting epoxy resin are unlikely to react with each other, so that the molecular weight-increasing reaction can be easily controlled, which is preferred. Meanwhile, when the amount of the alkali metal hydroxide is equal to or less than the above-described upper limit, the generation of impurities by a side reaction is unlikely to occur, which is preferred. The alkali metal hydroxide used here is usually, for example, sodium hydroxide or potassium hydroxide.

This reaction can be carried out under normal pressure or reduced pressure, and the reaction temperature is preferably 20 to 150°C, more preferably 30 to 100°C. When the reaction temperature is equal to or higher than the above-described lower limit, the reaction is likely to proceed and can be easily controlled, which is preferred. Meanwhile, when the reaction temperature is equal to or lower than the above-described upper limit, a side reaction is unlikely to proceed, so that particularly chlorine impurities are likely to be reduced, which is preferred.

The reaction may be carried out while performing dehydration by a method in which the reaction solution is azeotropically distilled while maintaining a prescribed temperature as required, a condensate liquid obtained by cooling a volatile vapor is subjected to oil-water separation, and an oil component removed of water is returned to the reaction system. In order to inhibit a rapid reaction, the alkali metal hydroxide is added intermittently or continuously in small portions over a period of preferably 0.1 to 8 hours, more preferably 0.5 to 6 hours. When the duration of adding the alkali metal hydroxide is equal to or longer than the above-described lower limit, the reaction can be prevented from rapidly proceeding, so that the reaction temperature can be easily controlled, which is preferred. The duration of adding the alkali metal hydroxide is preferably not longer than the above-described upper limit since this is likely to make the generation of chlorine impurities less likely to occur, and this is also preferred from the standpoint of economic efficiency. After the completion of the reaction, insoluble by-product salts are removed by filtration or washing with water, and unreacted epihalohydrin is subsequently removed by vacuum distillation, whereby a crude epoxy resin can be obtained.

In this reaction, a catalyst, examples of which include quaternary ammonium salts such as tetramethylammonium chloride and tetraethylammonium bromide, tertiary amines such as benzyldimethylamine and 2,4,6-tris(dimethylaminomethyl)phenol, imidazoles such as 2-ethyl-4-methylimidazole and 2-phenylimidazole, phosphonium salts such as ethyltriphenylphosphonium iodide, and phosphines such as triphenyl phosphine, may be used as well.

Further, in this reaction, an inert organic solvent, examples of which include alcohols such as ethanol and isopropanol, ketones such as acetone, methyl ethyl ketone, and methyl isobutyl ketone, ethers such as dioxane and ethylene glycol dimethyl ether, glycol ethers such as methoxypropanol, and polar aprotic solvents such as dimethylsulfoxide and dimethylformamide, may be used.

The crude epoxy resin produced in the above-described manner is again purified by reaction with an alkali metal hydroxide, whereby the epoxy resin of the present embodiment can be obtained.

The conditions of an alkali treatment for the production of the epoxy resin of the present embodiment will now be described; however, since the reaction rate is variable depending on the conditions, the desired epoxy resin can be obtained by taking samples at appropriate timings during the reaction and analyzing the epoxy equivalent thereof.

In the reaction of the epoxy resin and the alkali metal hydroxide, an organic solvent for dissolving the epoxy resin may be used. The organic solvent used in the reaction is not particularly limited; however, from the standpoint of the production efficiency, the ease of handling, the workability, and the like, it is preferred to use a ketone-based organic solvent. From the standpoint of further reducing the amount of hydrolyzable chlorine, a polar aprotic solvent may be used as well.

Examples of the ketone-based organic solvent include ketone-based solvents, such as methyl ethyl ketone, methyl isobutyl ketone, and cyclohexanone. Methyl isobutyl ketone is particularly preferred from the standpoint of the ease of post-treatment and the like. These ketone-based solvents may be used singly, or in combination of two or more thereof as a mixture.

Examples of the polar aprotic solvent include dimethyl sulfoxide, diethyl sulfoxide, dimethyl sulfone, sulfolane, dimethylformamide, dimethylacetamide, and hexamethylphosphoramide. These polar aprotic solvents may be used singly, or in combination of two or more kinds thereof as a mixture. Among these polar aprotic solvents, dimethyl sulfoxide is preferred since it is readily available and likely to reduce the amount of hydrolyzable chlorine.

In the case of using a mixture of the above-described ketone organic solvent and polar aprotic solvent, the polar aprotic solvent is used at a ratio of 1% to 80% by weight, preferably 5 to 30% by weight, with respect to a total amount of these solvents.

The amount of the solvent to be used is such an amount that the concentration of the epoxy resin in a liquid treated with the alkali metal hydroxide is usually 1 to 90% by weight, preferably 5 to 80% by weight.

The alkali metal hydroxide can be used in the form of a solid or a solution. Examples of the alkali metal hydroxide include potassium hydroxide and sodium hydroxide, and the alkali metal hydroxide is preferably sodium hydroxide. The alkali metal hydroxide may be used in the form of being dissolved in an organic solvent or water. The alkali metal oxide is preferably used as a solution in which the alkali metal oxide is dissolved in a water solvent or an organic solvent.

The amount of the alkali metal hydroxide to be used is preferably 0.1 parts by weight to 20 parts by weight with respect to 100 parts by weight of the epoxy resin in terms of the solid content of the alkali metal hydroxide. By controlling the amount of the alkali metal hydroxide to be in this range, the epoxy equivalent of the resulting epoxy resin can be adjusted. When the amount of the alkali metal hydroxide is in this range, the epoxy resin of the present embodiment is likely to be obtained.

The reaction temperature is preferably 10 to 150°C, more preferably 20 to 90°C, and the reaction time is preferably 0.1 to 15 hours, more preferably 0.3 to 10 hours. When the reaction temperature is in this range, the epoxy resin of the present embodiment is likely to be obtained.

After the reaction, excess alkali metal hydroxide and by-product salts are removed by a method such as washing with water, and the organic solvent is subsequently removed by vacuum distillation and/or steam distillation, whereby the epoxy resin of the present embodiment can be obtained.

### [Epoxy Resin Composition]

The epoxy resin composition according to another embodiment of the present invention contains at least the above-described epoxy resin according to one embodiment of the present invention and a curing agent. In the epoxy resin composition of the present embodiment, if necessary, an epoxy resin other than the epoxy resin of the present embodiment (hereinafter, may be simply referred to as "other epoxy resin"), a curing accelerator, an inorganic filler, a coupling agent, and the like may be incorporated as appropriate.

The epoxy resin composition of the present embodiment that contains the above-described epoxy resin yields a cured product sufficiently satisfying the physical properties required for various applications, such as excellent heat resistance.

### [Curing Agent]

In the present invention, the term "curing agent" refers to a substance that contributes to a crosslinking reaction and/or a chain extension reaction between epoxy groups of the epoxy resin. It is noted here that, in the present invention, even what is usually called a "curing accelerator" is regarded as a curing agent as long as it is a substance that contributes to a crosslinking reaction and/or a chain extension reaction between epoxy groups of the epoxy resin.

In the epoxy resin composition of the present embodiment, the content of the curing agent is preferably 0.1 to 1,000 parts by weight, more preferably 500 parts by weight or less, still more preferably 300 parts by weight or less, with respect to 100 parts by weight of all epoxy resin components as a solid content. In the present invention, the term "solid content" refers to components excluding solvents, and includes not only a solid epoxy resin but also a semisolid and a viscous liquid. Further, the amount of "all epoxy resin components" is the amount of epoxy resins contained in the epoxy resin composition of the present embodiment. When the epoxy resin composition of the present embodiment contains only the epoxy resin according to one embodiment of the present invention, the amount of "all epoxy resin components" corresponds to the amount of this epoxy resin, while when the epoxy resin composition of the present embodiment contains the epoxy resin according to one embodiment of the present invention and other epoxy resin, the amount of "all epoxy resin components" corresponds a total amount of the epoxy resin according to one embodiment of the present invention and the other epoxy resin.

The curing agent is not particularly limited, and any curing agent generally known as an epoxy resin curing agent can be used. Examples thereof include: a phenolic curing agent; an amine-based curing agent (excluding a tertiary amine), such as an aliphatic amine, a polyether amine, an alicyclic amine, and an aromatic amine; a tertiary amine; an acid anhydride-based curing agent; an amide-based curing agent; and an imidazole.

There among, the curing agent is preferably a phenolic curing agent since the epoxy resin composition of the present embodiment can attain excellent heat resistance, stress resistance, moisture absorption resistance, flame retardancy, and the like by containing a phenolic curing agent. From the standpoint of the heat resistance and the like, the epoxy resin composition of the present embodiment preferably contains an acid anhydride-based curing agent or an amide-based curing agent. It is also preferred to use an imidazole from the standpoint of allowing the curing reaction to proceed sufficiently and improving the heat resistance.

The curing agent may be used singly, or in combination of two or more kinds thereof. When two or more kinds of curing agents are used in combination, they may be mixed in advance to prepare a curing agent mixture before use, or each of the curing agents may be separately added and mixed simultaneously at the time of mixing the components of the epoxy resin composition.

### <Phenolic Curing Agent>

Specific examples of the phenolic curing agent include: various polyhydric phenols, such as bisphenol A, bisphenol F, bisphenol S, bisphenol AD, hydroquinone, resorcin, methyl resorcin, biphenol, tetramethylbiphenol, dihydroxynaphthalene, dihydroxydiphenyl ether, thiodiphenols, phenol novolac resins, cresol novolac resins, phenol aralkyl resins, biphenyl aralkyl resins, naphthol aralkyl resins, terpene phenol resins, dicyclopentadiene phenol resins, bisphenol A novolac resins, trisphenol methane-type resins, naphthol novolac resins, brominated bisphenol A, and brominated phenol novolac resins; and various phenolic resins, such as polyhydric phenolic resins obtained by condensation reaction of a phenol and an aldehyde such as benzaldehyde, hydroxybenzaldehyde, crotonaldehyde, or glyoxal, polyhydric phenolic resins obtained by condensation reaction of a xylene resin and a phenol, and co-condensation resins of a heavy oil or a pitch, a phenol, and a formaldehyde, as well as phenol-benzaldehyde-xylylene dimethoxide polycondensates, phenol-benzaldehyde-xylylene dihalide polycondensates, phenol-benzaldehyde-4,4'-dimethoxide biphenyl polycondensates, and phenol-benzaldehyde-4,4'-dihalide biphenyl polycondensates.

These phenolic curing agents may be used singly, or in any combination of two or more thereof at any blending ratio.

Among the above-exemplified phenolic curing agents, from the standpoint of the post-curing heat resistance, the curability, and the like of the composition, for example, phenol novolac resins (e.g., compounds represented by the following Formula (1)), phenol aralkyl resins (e.g., compounds represented by the following Formula (2)), biphenyl aralkyl resins (e.g., compounds represented by the following Formula (3)), naphthol novolac resins (e.g., compounds represented by the following Formula (4)), naphthol aralkyl resins (e.g., compounds represented by the following Formula (5)), trisphenol methane-type resins (e.g., compounds represented by the following Formula (6)), phenol-benzaldehyde-xylylene dimethoxide polycondensates (e.g., compounds represented by the following Formula (7)), phenol-benzaldehyde-xylylene dihalide polycondensates (e.g., compounds represented by the following Formula (7)), phenol-benzaldehyde-4,4'-dimethoxide biphenyl polycondensates (e.g., compounds represented by the following Formula (8)), and phenol-benzaldehyde-4,4'-dihalide biphenyl polycondensates (e.g., compounds represented by the following Formula (8)) are preferred, and phenol novolac resins (e.g., compounds represented by the following Formula (1)), phenol aralkyl resins (e.g., compounds represented by the following Formula (2)), biphenyl aralkyl resins (e.g., compounds represented by the following Formula (3)), phenol benzaldehyde-xylylene dimethoxide polycondensates (e.g., compounds represented by the following Formula (7)), phenol-benzaldehyde-xylylene dihalide polycondensates (e.g., compounds represented by the following Formula (7)), phenol-benzaldehyde-4,4'-dimethoxide biphenyl polycondensates (e.g., compounds represented by the following Formula (8)), and phenol-benzaldehyde-4,4'-dihalide biphenyl polycondensates (e.g., compounds represented by the following Formula (8)) are particularly preferred. 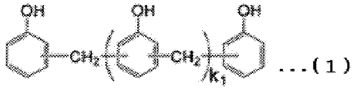 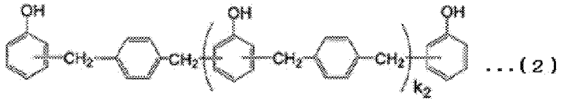 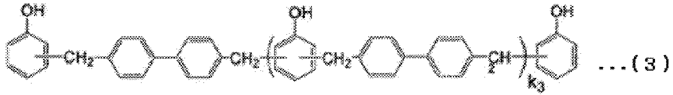 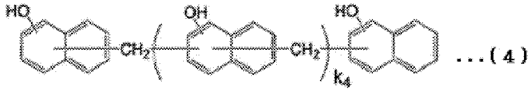 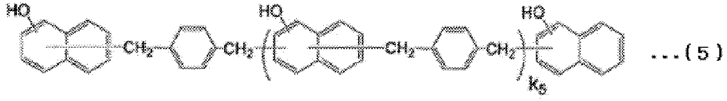 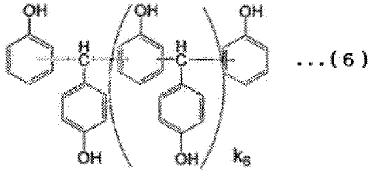 (in Formulae (1) to (6), k₁ to k₆ each represent an integer of 0 or larger) 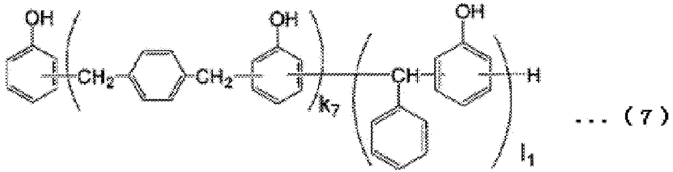 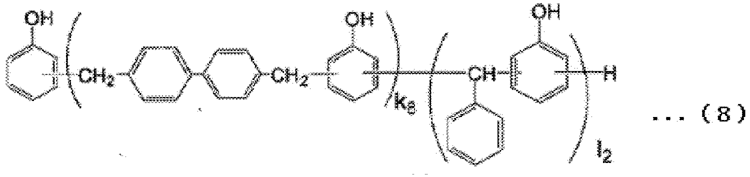 (in Formulae (7) and (8), k₇, k₈, l₁, and l₂ each represent an integer of 1 or larger)

The amount of the phenolic curing agent to be incorporated is preferably 0.1 to 1,000 parts by weight, more preferably 500 parts by weight or less, still more preferably 300 parts by weight or less, particularly preferably 100 parts by weight or less, with respect to 100 parts by weight of all epoxy resin components in the epoxy resin composition.

### <Amine-Based Curing Agent>

Examples of the amine-based curing agent (excluding tertiary amines) include aliphatic amines, polyether amines, alicyclic amines, and aromatic amines.

Examples of the aliphatic amines include ethylenediamine, 1,3-diaminopropane, 1,4-diaminopropane, hexamethylenediamine, 2,5-dimethylhexamethylenediamine, trimethylhexamethylenediamine, diethylenetriamine, iminobispropylamine, bis(hexamethylene)triamine, triethylenetetramine, tetraethylenepentamine, pentaethylenehexamine, N-hydroxyethyl ethylenediamine, and tetra(hydroxyethyl) ethylenediamine.

Examples of the polyether amines include triethylene glycol diamine, tetraethylene glycol diamine, diethylene glycol bis(propylamine), polyoxypropylene diamine, and polyoxypropylene triamine.

Examples of the alicyclic amines include isophoronediamine, menthenediamine, N-aminoethylpiperazine, bis(4-amino-3-methyldicyclohexyl)methane, bis(aminomethyl)cyclohexane, 3,9-bis(3-aminopropyl)-2,4,8,10-tetraoxaspiro(5,5)undecane, and norbornenediamine.

Examples of the aromatic amines include tetrachloro-p-xylenediamine, m-xylenediamine, p-xylenediamine, m-phenylenediamine, o-phenylenediamine, p-phenylenediamine, 2,4-diaminoanisole, 2,4-toluenediamine, 2,4-diaminodiphenylmethane, 4,4'-diaminodiphenylmethane, 4,4'-diamino-1,2-diphenylethane, 2,4-diaminodiphenyl sulfone, 4,4'-diaminodiphenyl sulfone, m-aminophenol, m-aminobenzylamine, benzyldimethylamine, 2-(dimethylaminomethyl)phenol, triethanolamine, methylbenzylamine, α-(m-aminophenyl)ethylamine, α-(p-aminophenyl)ethylamine, diaminodiethyldimethyldiphenylmethane, α,α'-bis(4-aminophenyl)-p-diisopropylbenzene.

The above-exemplified amine-based curing agents may be used singly, or in any combination of two or more thereof at any blending ratio.

The amine-based curing agents are each preferably used such that the equivalent ratio of the functional groups in the curing agent with respect to the epoxy groups in all epoxy resin components contained in the epoxy resin composition is in a range of 0.8 to 1.5. In this range, unreacted epoxy groups and the functional groups of the curing agent are unlikely to remain, which is preferred.

Examples of the tertiary amines include 1,8-diazabicyclo(5,4,0)undesen-7, triethylenediamine, benzyldimethylamine, triethanolamine, dimethylaminoethanol, and tris(dimethylaminomethyl)phenol.

The above-exemplified tertiary amines may be used singly, or in any combination of two or more thereof at any blending ratio.

The tertiary amines are each preferably used such that the equivalent ratio of the functional groups in the curing agent with respect to the epoxy groups in all epoxy resin components contained in the epoxy resin composition is in a range of 0.8 to 1.5. In this range, unreacted epoxy groups and the functional groups of the curing agent are unlikely to remain, which is preferred.

### <Acid Anhydride-Based Curing Agents>

Examples of the acid anhydride-based curing agents include acid anhydrides and modified acid anhydrides.

Examples of the acid anhydrides include phthalic anhydride, trimellitic anhydride, pyromellitic anhydride, benzophenone tetracarboxylic anhydride, dodecenyl succinic anhydride, polyadipic anhydride, polyazelaic anhydride, polysebacic anhydride, poly(ethyloctadecane diacid) anhydride, poly(phenylhexadecane diacid) anhydride, tetrahydrophthalic anhydride, methyltetrahydrophthalic anhydride, methylhexahydrophthalic anhydride, hexahydrophthalic anhydride, methylhimic anhydride, trialkyltetrahydrophthalic anhydride, methylcyclohexene dicarboxylic anhydride, methylcyclohexene tetracarboxylic anhydride, ethylene glycol bistrimellitate dianhydride, HET anhydride, nadic anhydride, methyl nadic anhydride, 5-(2,5-dioxotetrahydro-3-furanyl)-3-methyl-3-cyclohexane-1,2-dicarboxylic anhydride, and 3,4-dicarboxy-1,2,3,4-tetrahydro-1-naphthalene succinic dianhydride.

Examples of the modified acid anhydrides include products obtained by modification of the above-exemplified acid anhydrides with a glycol. Examples of the glycol that can be used for the modification include: alkylene glycols, such as ethylene glycol, propylene glycol, and neopentyl glycol; and polyether glycols, such as polyethylene glycol, polypropylene glycol, and polytetramethylene ether glycol. Further, a copolymer polyether glycol of two or more kinds of these glycols and/or polyether glycols can be used as well.

In the modified acid anhydrides, it is preferred that 1 mol of an acid anhydride be modified with 0.4 mol or less of a glycol. When the amount of modification is equal to or less than this upper limit value, not only the epoxy resin composition does not have an excessively high viscosity and thus tends to have favorable workability, but also the rate of the curing reaction with the epoxy resin tends to be favorable.

The above-exemplified acid anhydride-based curing agents may be used singly, or in any combination of two or more thereof in any amount.

In the case of using any of the acid anhydride-based curing agents, it is preferably used such that the equivalent ratio of the functional groups in the curing agent with respect to the epoxy groups in all epoxy resin components contained in the epoxy resin composition is in a range of 0.8 to 1.5. In this range, unreacted epoxy groups and the functional groups of the curing agents are unlikely to remain, which is preferred.

### <Amide-Based Curing Agent>

Examples of the amide-based curing agent include dicyandiamide, derivatives thereof, and polyamide resins.

These amide-based curing agents may be used singly, or in any combination of two or more thereof at any ratio.

In the case of using any of the amide-based curing agents, it is preferably used in an amount of 0.1 to 20% by weight with respect to a total amount of all epoxy resin components contained in the epoxy resin composition and the amide-based curing agent.

### <Imidazole>

Examples of the imidazole include 2-phenylimidazole, 2-ethyl-4(5)-methylimidazole, 2-phenyl-4-methylimidazole, 1-benzyl-2-methylimidazole, 1-benzyl-2-phenylimidazole, 1-cyanoethyl-2-undecylimidazole, 1-cyano-2-phenylimidazole, 1-cyanoethyl-2-undecylimidazole trimellitate, 1-cyanoethyl-2-phenylimidazolium trimellitate, 2,4-diamino-6-[2'-methylimidazolyl-(1')]-ethyl-s-triazine, 2,4-diamino-6-[2'-ethyl-4'-methylimidazolyl-(1')]-ethyl-s-triazine, 2,4-diamino-6-[2'-methylimidazolyl-(1')]-ethyl-s-triazine isocyanuric acid adduct, 2-phenylimidazole isocyanuric acid adduct, 2-phenyl-4,5-dihydroxymethylimidazole, 2-phenyl-4-methyl-5-hydroxymethylimidazole, and adducts of an epoxy resin and the above-described imidazoles. These imidazoles have a catalytic ability and thus can also be generally classified as curing accelerators; however, in the present invention, they are classified as curing agents.

The above-exemplified imidazoles may be used singly, or in any combination of two or more thereof at any ratio.

In the case of using any of the imidazoles, it is preferably used in an amount of 0.1 to 20% by weight with respect to a total amount of all epoxy resin components contained in the epoxy resin composition and the imidazole.

### <Other Curing Agent>

The epoxy resin composition of the present embodiment may also contain other curing agent in addition to the above-described curing agent. The other curing agent that can be used in the epoxy resin composition of the present embodiment is not particularly limited, and any curing agent generally known as an epoxy resin curing agent can be used.

Such other curing agent may be used singly, or in combination of two or more thereof.

### [Other Epoxy Resin]

The epoxy resin composition of the present embodiment may further contain other epoxy resin in addition to the epoxy resin of the present embodiment. By incorporating other epoxy resin, the heat resistance, the stress resistance, the moisture absorption resistance, the flame retardancy, and the like of the epoxy resin composition of the present embodiment can be improved.

Epoxy resins other than the epoxy resin of the present embodiment are all relevant as the other epoxy resin that can be used in the epoxy resin composition of the present embodiment, and specific examples thereof include bisphenol A-type epoxy resins, trisphenol methane-type epoxy resins, anthracene-type epoxy resins, phenol-modified xylene resin-type epoxy resins, bisphenol cyclododecyl-type epoxy resins, bisphenol diisopropylidene resorcinol-type epoxy resins, bisphenol F-type epoxy resins, bisphenol AD-type epoxy resins, hydroquinone-type epoxy resins, methylhydroquinone-type epoxy resins, dibutylhydroquinone-type epoxy resins, resorcinol-type epoxy resins, methylresorcinol-type epoxy resins, biphenol-type epoxy resins, tetramethylbiphenol-type epoxy resins other than the epoxy resin of the present embodiment, tetramethylbisphenol F-type epoxy resins, dihydroxydiphenyl ether-type epoxy resins, epoxy resins derived from thiodiphenols, dihydroxynaphthalene-type epoxy resins, dihydroxyanthracene-type epoxy resins, dihydroxydihydroanthracene-type epoxy resins, dicyclopentadiene-type epoxy resins, epoxy resins derived from dihydroxystilbenes, phenol novolac-type epoxy resins, cresol novolac-type epoxy resins, bisphenol A novolac-type epoxy resins, naphthol novolac-type epoxy resins, phenol aralkyl-type epoxy resins, naphthol aralkyl-type epoxy resins, biphenylaralkyl-type epoxy resins, terpene phenol-type epoxy resins, dicyclopentadiene phenol-type epoxy resins, epoxy resins derived from a phenol-hydroxybenzaldehyde condensate, epoxy resins derived from a phenol-crotonaldehyde condensate, epoxy resins derived from a phenol-glyoxal condensate, epoxy resins derived from a co-condensation resin of a phenol and formaldehyde with a heavy oil or a pitch, epoxy resins derived from diaminodiphenylmethane, epoxy resins derived from aminophenols, epoxy resins derived from xylenediamine, epoxy resins derived from methylhexahydrophthalic acid, and epoxy resins derived from dimer acids.

These epoxy resins may be used singly, or in any combination of two or more thereof at any blending ratio.

Among the above-exemplified epoxy resins, from the standpoint of the fluidity of the composition as well as the heat resistance, the moisture absorption resistance, the flame retardancy, and the like of a cured product, bisphenol A-type epoxy resins, tetramethylbiphenol-type epoxy resins other than the epoxy resin according to one embodiment of the present invention, 4,4'-biphenol-type epoxy resins, biphenyl aralkyl-type epoxy resins, phenol aralkyl-type epoxy resins, dihydroxyanthracene-type epoxy resins, dicyclopentadiene-type epoxy resins, ortho-cresol novolac-type epoxy resins, and trisphenol methane-type epoxy resins are particularly preferred.

When the epoxy resin composition of the present embodiment contains the above-described other epoxy resin, the content thereof is preferably 0.01 to 60 parts by weight, more preferably 40 parts by weight or less, still more preferably 30 parts by weight or less, particularly preferably 20 parts by weight or less, but more preferably 1 part by weight or more, with respect to 100 parts by weight of all epoxy resin components contained in the composition.

### [Curing Accelerator]

The epoxy resin composition of the present embodiment preferably contains a curing accelerator. By incorporating a curing accelerator, the curing time can be shortened and the curing temperature can be lowered, so that the desired cured product can be easily obtained.

Specific examples of the curing accelerator include, but not particularly limited to: phosphorus compounds, such as organophosphines and phosphonium salts; tetraphenylboron salts; organic acid dihydrazides; and boron halide amine complexes.

Examples of phosphorus compounds that can be used as the curing accelerator include: organophosphines, such as triphenyl phosphine, diphenyl(p-tolyl)phosphine, tris(alkylphenyl)phosphine, tris(alkoxyphenyl)phosphine, tris(alkylalkoxyphenyl)phosphine, tris(dialkylphenyl)phosphine, tris(trialkylphenyl)phosphine, tris(tetraalkylphenyl)phosphine, tris(dialkoxyphenyl)phosphine, tris(trialkoxyphenyl)phosphine, tris(tetraalkoxyphenyl)phosphine, trialkyl phosphine, dialkylaryl phosphine, and alkyldiaryl phosphine; complexes of these organophosphines with organoborons; and compounds obtained by addition of any of the above-exemplified organophosphines with maleic anhydride, a quinone compound such as 1,4-benzoquinone, 2,5-toluquinone, 1,4-naphthoquinone, 2,3-dimethylbenzoquinone, 2,6-dimethylbenzoquinone, 2,3-dimethoxy-5-methyl-1,4-benzoquinone, 2,3-dimethoxy-1,4-benzoquinone, or phenyl-1,4-benzoquinone, or a compound such as diazophenylmethane.

Among the above-exemplified curing accelerators, organophosphines and phosphonium salts are preferred, and organophosphines are most preferred. Further, the above-exemplified curing accelerators may be used singly, or in any combination of two or more thereof at any ratio as a mixture.

The curing accelerator is preferably used in a range of 0.1 parts by weight to 20 parts by weight, more preferably 0.5 parts by weight or more, still more preferably 1 part by weight or more, but more preferably 15 parts by weight or less, still more preferably 10 parts by weight or less, with respect to 100 parts by weight of all epoxy resin components contained in the epoxy resin composition. When the content of the curing accelerator is equal to or more than the above-described lower limit value, a good curing acceleration effect can be obtained, while when the content of the curing accelerator is equal to or less than the above-described upper limit value, desired curing properties are likely to be obtained, which is preferred.

### [Inorganic Filler]

An inorganic filler may be incorporated into the epoxy resin composition of the present embodiment. Examples of the inorganic filler include fused silica, crystalline silica, glass powder, alumina, calcium carbonate, calcium sulfate, talc, and boron nitride. These inorganic fillers may be used singly, or in any combination of two or more thereof at any blending ratio. Thereamong, when the epoxy resin composition is used for semiconductor sealing, a crushed and/or spherical, fused and/or crystalline silica powder filler is preferred.

When the epoxy resin composition is used as a semiconductor sealing material, the use of such an inorganic filler can not only bring the thermal expansion coefficient of the semiconductor sealing material close to those of an internal silicon chip and a lead frame, but also reduce the amount of moisture absorbed by the semiconductor sealing material as a whole, so that the solder crack resistance can be improved.

The inorganic filler has an average particle size of 0.01 to 50 um, preferably 1 to 40 um, more preferably 2 to 30 um. When the average particle size is equal to or larger than the lower limit value, the melt viscosity is not excessively high, and the fluidity is thus unlikely to be reduced, which is preferred. When the average particle size is equal to or smaller than the upper limit value, the filler is unlikely to cause clogging of a narrow gap of a mold during molding, and the filling property of materials is thus likely to be improved, which is preferred.

When an inorganic filler is used in the epoxy resin composition of the present embodiment, the inorganic filler is preferably incorporated in a range of 60 to 95% by weight of the whole epoxy resin composition.

### [Mold Release Agent]

A mold release agent may be incorporated into the epoxy resin composition of the present embodiment. Examples of the mold release agent that can be used include: natural waxes, such as carnauba wax; synthetic waxes, such as polyethylene wax; higher fatty acids and metal salts thereof, such as stearic acid and zinc stearate; and hydrocarbon-based mold release agents, such as paraffin. These mold release agents may be used singly, or in any combination of two or more thereof at any blending ratio.

When a mold release agent is incorporated into the epoxy resin composition of the present embodiment, the amount of the mold release agent is preferably 0.1 to 5.0 parts by weight, more preferably 0.5 to 3.0 parts by weight, with respect to 100 parts by weight of all epoxy resin components contained in the epoxy resin composition. When the amount of the mold release agent is in this range, good mold releasability can be exerted while the curing properties of the epoxy resin composition are maintained, which is preferred.

### [Coupling Agent]

It is preferred to incorporate a coupling agent into the epoxy resin composition of the present embodiment. The coupling agent is preferably used in combination with an inorganic filler and, by incorporating the coupling agent, the adhesion between the epoxy resin serving as a matrix and the inorganic filler can be improved. The coupling agent is, for example, a silane coupling agent or a titanate coupling agent.

Examples of the silane coupling agent include: epoxysilanes, such as γ-glycidoxypropyltrimethoxysilane, γ-glycidoxypropyltriethoxysilane, and β-(3,4-epoxycyclohexyl)ethyltrimethoxysilane; aminosilanes, such as γ-aminopropyltriethoxysilane, N-β(aminoethyl)-γ-aminopropyltrimethoxysilane, N-β(aminoethyl)-γ-aminopropylmethyldimethoxysilane, γ-aminopropyltrimethoxysilane, and γ-ureidopropyltriethoxysilane; mercaptosilanes, such as 3-mercaptopropyltrimethoxysilane; vinylsilanes, such as p-styryltrimethoxysilane, vinyltrichlorosilane, vinyltris(β-methoxyethoxy)silane, vinyltrimethoxysilane, vinyltriethoxysilane, and γ-methacryloxypropyltrimethoxysilane; and epoxy-based, amino-based, and vinyl-based polymer-type silanes.

Examples of the titanate coupling agent include isopropyl triisostearoyl titanate, isopropyl tri(N-aminoethyl-aminoethyl) titanate, diisopropyl bis(dioctylphosphate) titanate, tetraisopropyl bis(dioctylphosphite) titanate, tetraoctyl bis(ditridecylphosphite) titanate, tetra(2,2-diallyloxymethyl-1-butyl) bis(ditridecyl)phosphite titanate, bis(dioctylpyrophosphate) oxyacetate titanate, and bis(dioctylpyrophosphate) ethylene titanate.

These coupling agents may be used singly, or in any combination of two or more thereof at any ratio.

When a coupling agent is used in the epoxy resin composition of the present embodiment, the amount of the coupling agent to be incorporated is preferably 0.1 to 3.0 parts by weight with respect to 100 parts by weight of all epoxy resin components. When the amount of the coupling agent is equal to or greater than the above-described lower limit value, the effect of improving the adhesion between the epoxy resin serving as a matrix and the inorganic filler, which effect is obtained by incorporating the coupling agent, tends to be improved, while when the amount of the coupling agent is equal to or less than the above-described upper limit value, the coupling agent is unlikely to bleed out from the resulting cured product, which is preferred.

### [Other Components]

In the epoxy resin composition of the present embodiment, components other than the above-described ones (hereinafter, may be referred to as "other components") may be incorporated. Examples of the other components include flame retardants, plasticizers, reactive diluents, and pigments, which may be incorporated as appropriate if necessary. It is noted that the epoxy resin composition of the present embodiment is not restricted from containing a component other than the above-described ones.

Examples of the flame retardants that may be used in the epoxy resin composition of the present embodiment include: halogen-based flame retardants, such as brominated epoxy resins and brominated phenol resins; antimony compounds, such as antimony trioxide; phosphorus-based flame retardants, such as red phosphorus, phosphates, and phosphines; nitrogen-based flame retardants, such as melamine derivatives; and inorganic flame retardants, such as aluminum hydroxide and magnesium hydroxide.

### [Cured Product]

The epoxy resin composition according to one embodiment of the present invention can be cured to obtain a cured product having excellent heat resistance. In other words, another embodiment of the present invention is a cured product obtained by curing the epoxy resin composition according to one embodiment of the present invention.

A method of curing the epoxy resin composition is not particularly limited and, usually, a cured product can be obtained through a thermosetting reaction by heating. During the thermosetting reaction, it is preferred to select the curing temperature as appropriate in accordance with the type of a curing agent being used. For example, when a phenolic curing agent is used, the curing temperature is usually 100 to 300°C. Further, by adding a curing accelerator to the curing agent, the curing temperature can be lowered. The reaction time is preferably 0.01 to 20 hours, more preferably 0.1 to 10 hours. When the reaction time is equal to or longer than the above-described lower limit value, the curing reaction tends to proceed sufficiently, which is preferred. Meanwhile, when the reaction time is equal to or shorter than the above-described upper limit value, deterioration caused by the heating and an energy loss during the heating are likely to be reduced, which is preferred.

The cured product of the present embodiment has excellent heat resistance. The cured product preferably has a glass transition temperature (Tg) of 130°C or higher, and a 5% weight reduction temperature of 390°C or higher. The higher the glass transition temperature and the 5% weight reduction temperature of the cured product, the more likely is that the physical properties can be maintained even in a high-ambient temperature condition, which is preferred.

It is noted here that the glass transition temperature (Tg) and the 5% weight reduction temperature are measured by the respective methods described below in the section of Examples.

### [Use]

The cured product of the present embodiment has excellent heat resistance; therefore, the phenol resin composition, the epoxy resin, the epoxy resin composition, and the cured product according to the above-described respective embodiments of the present invention can be effectively used in any application where such physical property is required. They can be suitably used in any application in, for example: the field of paints, such as electrodeposition paints for automobiles, heavy-duty anti-corrosion paints for ships and bridges, and paints for inner coating of beverage cans; the electrical and electronic fields, such as laminates, semiconductor sealing materials, insulating powder paints, and coil impregnation; the fields of civil engineering, construction, and adhesives, such as seismic reinforcement of bridges, concrete reinforcement, flooring materials for buildings, linings for water supply facilities, drainage and permeable pavement, and adhesives for vehicles and airplanes. Thereamong, the phenol resin composition, the epoxy resin, the epoxy resin composition, and the cured product according to the above-described respective embodiments of the present invention are particularly useful for electrical/electronic components such as semiconductor sealing materials and laminates.

The epoxy resin composition according to one embodiment of the present invention may be used in any of the above-described applications after being cured, or may be cured in the production process for any of the above-described applications.

### EXAMPLES

The present invention will now be described more concretely by way of Examples thereof; however, the present invention is not limited by the below-described Examples. The values of various production conditions and evaluation results in the below-described Examples each have a meaning as a preferred upper or lower limit value in an embodiment of the present invention, and a preferred range may be defined by a combination of an upper or lower limit value described above and a value described below in an Example, or a combination of values in Examples.

### [Measurement and Evaluation Methods]

### <Formulation of Phenol Mixture>

(1) Analysis of 2,6-xylenol (XNL) and 4-(2,6-dimethylphenoxy)-2,6-dimethylphenol (ED):
   Gas chromatography was employed for quantification. Using a phenylmethyl silicone capillary column having a crosslinking degree of 5% ("CBP-5", manufactured by J&W Scientific, Inc.) as a column, a flame ionization detector (FID) as a detector, and diphenyl as an internal standard substance, a measurement sample was prepared by dissolving 2 g of a sample in 25 mL of acetone.
(2) Analysis of Tetramethyldiphenoquinone (DPQ):
   Spectrophotometry was employed for quantification. The cell length and the measurement wavelength were 10 mm and 420 nm, respectively, and a measurement sample was prepared by dissolving 0.5 g of a sample in 100 mL of chloroform at constant volume.
(3) Analysis of Polyphenylene Ether (PPE) and 3,3',5,5'-tetramethyl-4,4'-biphenol (TMBPL):
   Liquid chromatography was employed for quantification. A "SHODEX KF-801", manufactured by Showa Denko K.K. (exclusion limit molecular weight: about 1,500) was used as a molecular sieve column, tetrahydrofuran was used as a carrier (mobile phase), a UV detector (268 nm) was used as a detector, and benzene was used as an internal standard substance. A measurement sample was prepared by dissolving 50 mg of a sample in 5 mL of tetrahydrofuran.

### [Production of Phenol Mixtures]

### [Example 1]

To a 30-L stainless steel reactor equipped with a baffle plate, 4 kg of 2,6-xylenol (XNL), 400 g of borax, 13 g of sodium lauryl sulfate, 12 kg of water, and 0.2 g of copper (II) acetate were added, and these materials were heated with stirring. Once the temperature of the contents reached 70°C, introduction of 0.26 kg of oxygen was initiated. Stirring was continued such that the reaction temperature was maintained at 70°C, and the introduction of oxygen was terminated after 12 hours, followed by purging of the reaction system with nitrogen. Subsequently, after adjusting the pH of the resulting slurry to be 7.5 with an addition of 25% sulfuric acid, the temperature was gradually increased to remove water and unreacted 2,6-xylenol by distillation. Then, the temperature inside the reactor was cooled to 70°C, and the pH of the reaction solution was adjusted to be 6.5 with an addition of 25% sulfuric acid, after which 1.75 kg of water and 6.05 kg of isopropyl alcohol were added, and the resultant was stirred for 30 minutes with the temperature being maintained at 60°C. Thereafter, the temperature was maintained at 60°C, and the thus obtained slurry was subjected to a solid-liquid separation treatment using a centrifuge, and the resulting solid in the centrifuge was rinsed with 5 kg of 60°C hot water. The thus obtained solid was dried in a vacuum dryer, whereby a phenol mixture was obtained.

### [Example 2]

To a 30-L stainless steel reactor equipped with a baffle plate, 4 kg of 2,6-xylenol (XNL), 400 g of borax, 13 g of sodium lauryl sulfate, 12 kg of water, and 0.2 g of copper (II) acetate were added, and these materials were heated with stirring. Once the temperature of the contents reached 70°C, introduction of 0.27 kg of oxygen was initiated. Stirring was continued such that the reaction temperature was maintained at 70°C, and the introduction of oxygen was terminated after 12 hours, followed by purging of the reaction system with nitrogen. Subsequently, after adjusting the pH of the resulting slurry to be 7.8 with an addition of 25% sulfuric acid, the temperature was gradually increased to remove water and unreacted 2,6-xylenol by distillation. Then, the temperature inside the reactor was cooled to 70°C, and the pH of the reaction solution was adjusted to be 6.5 with an addition of 25% sulfuric acid, after which 1.75 kg of water and 6.05 kg of isopropyl alcohol were added, and the resultant was stirred for 30 minutes with the temperature being maintained at 60°C. Thereafter, the temperature was maintained at 60°C, and the thus obtained slurry was subjected to a solid-liquid separation treatment using a centrifuge, and the resulting solid in the centrifuge was rinsed with 5 kg of 60°C hot water. The thus obtained solid was dried in a vacuum dryer, whereby a phenol mixture was obtained.

### [Example 3]

To a 30-L stainless steel reactor equipped with a baffle plate, 4 kg of 2,6-xylenol (XNL), 400 g of borax, 13 g of sodium lauryl sulfate, 12 kg of water, and 0.2 g of copper (II) acetate were added, and these materials were heated with stirring. Once the temperature of the contents reached 70°C, introduction of 0.31 kg of oxygen was initiated. Stirring was continued such that the reaction temperature was maintained at 70°C, and the introduction of oxygen was terminated after 12 hours, followed by purging of the reaction system with nitrogen. Subsequently, after adjusting the pH of the resulting slurry to be 8.1 with an addition of 25% sulfuric acid, the temperature was gradually increased to remove water and unreacted 2,6-xylenol by distillation. Then, the temperature inside the reactor was cooled to 70°C, and the pH of the reaction solution was adjusted to be 6.5 with an addition of 25% sulfuric acid, after which 1.75 kg of water and 6.05 kg of isopropyl alcohol were added, and the resultant was stirred for 30 minutes with the temperature being maintained at 60°C. Thereafter, the temperature was maintained at 60°C, and the thus obtained slurry was subjected to a solid-liquid separation treatment using a centrifuge, and the resulting solid in the centrifuge was rinsed with 5 kg of 60°C hot water. The thus obtained solid was dried in a vacuum dryer, whereby a phenol mixture was obtained.

### [Example 4]

To a 30-L stainless steel reactor equipped with a baffle plate, 4 kg of 2,6-xylenol (XNL), 400 g of borax, 13 g of sodium lauryl sulfate, 12 kg of water, and 0.2 g of copper (II) acetate were added, and these materials were heated with stirring. Once the temperature of the contents reached 70°C, introduction of 0.40 kg of oxygen was initiated. Stirring was continued such that the reaction temperature was maintained at 70°C, and the introduction of oxygen was terminated after 12 hours, followed by purging of the reaction system with nitrogen. Subsequently, after adjusting the pH of the resulting slurry to be 8.5 with an addition of 25% sulfuric acid, the temperature was gradually increased to remove water and unreacted 2,6-xylenol by distillation. Then, the temperature inside the reactor was cooled to 70°C, and the pH of the reaction solution was adjusted to be 6.5 with an addition of 25% sulfuric acid, after which 1.75 kg of water and 6.05 kg of isopropyl alcohol were added, and the resultant was stirred for 30 minutes with the temperature being maintained at 60°C. Thereafter, the temperature was maintained at 60°C, and the thus obtained slurry was subjected to a solid-liquid separation treatment using a centrifuge, and the resulting solid in the centrifuge was rinsed with 5 kg of 60°C hot water. The thus obtained solid was dried in a vacuum dryer, whereby a phenol mixture was obtained.

### [Example 5]

To a 30-L stainless steel reactor equipped with a baffle plate, 4 kg of 2,6-xylenol (XNL), 400 g of borax, 13 g of sodium lauryl sulfate, 12 kg of water, and 0.2 g of copper (II) acetate were added, and these materials were heated with stirring. Once the temperature of the contents reached 70°C, introduction of 0.47 kg of oxygen was initiated. Stirring was continued such that the reaction temperature was maintained at 70°C, and the introduction of oxygen was terminated after 12 hours, followed by purging of the reaction system with nitrogen. Subsequently, after adjusting the pH of the resulting slurry to be 9.0 with an addition of 25% sulfuric acid, the temperature was gradually increased to remove water and unreacted 2,6-xylenol by distillation. Then, the temperature inside the reactor was cooled to 70°C, and the pH of the reaction solution was adjusted to be 6.5 with an addition of 25% sulfuric acid, after which 1.75 kg of water and 6.05 kg of isopropyl alcohol were added, and the resultant was stirred for 30 minutes with the temperature being maintained at 60°C. Thereafter, the temperature was maintained at 60°C, and the thus obtained slurry was subjected to a solid-liquid separation treatment using a centrifuge, and the resulting solid in the centrifuge was rinsed with 5 kg of 60°C hot water. The thus obtained solid was dried in a vacuum dryer, whereby a phenol mixture was obtained.

### [Comparative Example 1]

A phenol mixture was obtained in the same manner as in Example 1 described in Japanese Unexamined Patent Application Publication No. 2003-327554.

To a 30-L stainless steel reactor equipped with a baffle plate, 4 kg of 2,6-xylenol (XNL), 400 g of borax, 13 g of sodium lauryl sulfate, and 12 kg of water were added, and these materials were heated with stirring. Once the temperature of the contents reached 70°C, 0.2 g of copper (II) acetate was added, and introduction of 0.21 kg of oxygen was initiated. Stirring was continued such that the reaction temperature was maintained at 70°C, and the introduction of oxygen was terminated after 12 hours, followed by purging of the reaction system with nitrogen. Subsequently, after adjusting the pH of the resulting slurry to be 6.5 with an addition of 25% sulfuric acid, the temperature was gradually increased to remove water and unreacted 2,6-xylenol by distillation. Then, the temperature inside the reactor was cooled to 70°C, and the pH of the resulting slurry was adjusted to be 3.8 with an addition of 25% sulfuric acid, after which 1.75 kg of water and 6.05 kg of isopropyl alcohol were added, and the resultant was stirred for 30 minutes with the temperature being maintained at 60°C. The thus obtained slurry was subjected to solid-liquid separation using a centrifuge, and the resulting solid in the centrifuge was rinsed with 5 kg of 60°C hot water. The thus obtained solid was dried in a vacuum dryer, whereby a phenol mixture was obtained.

### [Comparative Example 2]

A phenol mixture was obtained in the same manner as in Comparative Example 1 described in Japanese Unexamined Patent Application Publication No. 2004-002830.

To a 30-L stainless steel reactor equipped with a baffle plate, 4 kg of 2,6-xylenol (XNL), 400 g of borax, 13 g of sodium lauryl sulfate, and 12 kg of water were added, and these materials were heated with stirring. Once the temperature of the contents reached 70°C, 0.2 g of copper (II) acetate was added, and introduction of 0.21 kg of oxygen was initiated. Stirring was continued such that the reaction temperature was maintained at 70°C, and the introduction of oxygen was terminated after 12 hours, followed by purging of the reaction system with nitrogen. Subsequently, after adjusting the pH of the resulting slurry to be 6.5 with an addition of 25% sulfuric acid, the temperature was gradually increased to remove water and unreacted 2,6-xylenol by distillation. Then, the temperature inside the reactor was cooled to 70°C, and the pH of the resulting slurry was adjusted to be 3.8 with an addition of 25% sulfuric acid, after which 1.75 kg of water and 6.05 kg of isopropyl alcohol were added, and the resultant was stirred for 30 minutes with the temperature being maintained at 60°C. Thereafter, the temperature was maintained at 60°C, and the thus obtained slurry was subjected to a solid-liquid separation treatment using a centrifuge, and the resulting solid in the centrifuge was rinsed with 5 kg of 60°C hot water. The thus obtained solid was dried in a vacuum dryer to obtain a phenol resin. The thus obtained phenol resin in an amount of 1 kg along with 4.6 kg of isopropyl alcohol and 1.5 kg of water were added to a stirring tank, heated to 110°C under increased pressure, and stirred for 2 hours. Thereafter, the resultant was cooled to 30°C and subjected to solid-liquid separation using a centrifuge, after which 4.1 kg of isopropyl alcohol and 1.4 kg of water were added to the resulting solid and purification was performed again by the same method, whereby a phenol mixture was obtained.

### [Comparative Example 3]

A phenol mixture was obtained in the same manner as in Example 1 described in Japanese Unexamined Patent Application Publication No. S61-268641.

To a 10-L stainless steel reactor equipped with a baffle plate, 0.915 kg of 2,6-xylenol (XNL), 100 g of borax, 3 g of sodium lauryl sulfate, and 2.75 kg of water were added, and these materials were heated with stirring. Once the temperature of the contents reached 65°C, 0.06 g of copper (II) acetate was added, and introduction of 0.048 kg of oxygen was initiated. Stirring was continued such that the reaction temperature was maintained at 70°C, and the introduction of oxygen was terminated after 8 hours, followed by purging of the reaction system with nitrogen. Subsequently, the temperature inside the reactor was maintained at 70°C, and the pH of the resulting reaction solution was adjusted to be 6.5 with an addition of 25% sulfuric acid, after which 0.44 kg of water and 1.5 kg of isopropyl alcohol were added, and the resultant was stirred for 30 minutes with the temperature being maintained at 60°C. Thereafter, the temperature was maintained at 60°C, and the thus obtained slurry was subjected to a solid-liquid separation treatment using a centrifuge, and the resulting solid in the centrifuge was rinsed with 1 kg of 60°C hot water. The thus obtained solid was dried in a vacuum dryer, whereby a phenol mixture was obtained.

### [Comparative Example 4]

A phenol mixture was obtained in the same manner as in Example 2 described in Japanese Unexamined Patent Application Publication No. S61-268641.

To a 10-L stainless steel reactor equipped with a baffle plate, 0.915 kg of 2,6-xylenol (XNL), 60 g of sodium hydroxide, 3 g of sodium lauryl sulfate, and 2.75 kg of water were added, and these materials were heated with stirring. Once the temperature of the contents reached 65°C, 0.06 g of copper (II) acetate was added, and introduction of 0.048 kg of oxygen was initiated. Stirring was continued such that the reaction temperature was maintained at 70°C, and the introduction of oxygen was terminated after 8 hours, followed by purging of the reaction system with nitrogen. Subsequently, the temperature inside the reactor was maintained at 70°C, and the pH of the resulting reaction solution was adjusted to be 6.5 with an addition of 25% sulfuric acid, after which 0.44 kg of water and 1.5 kg of isopropyl alcohol were added, and the resultant was stirred for 30 minutes with the temperature being controlled at 60°C. Thereafter, the temperature was maintained at 60°C, and the thus obtained slurry was subjected to a solid-liquid separation treatment using a centrifuge, and the resulting solid in the centrifuge was rinsed with 1 kg of 60°C hot water. The thus obtained solid was dried in a vacuum dryer, whereby a phenol mixture was obtained.

### [Comparative Example 5]

To a 30-L stainless steel reactor equipped with a baffle plate, 4 kg of 2,6-xylenol (XNL), 400 g of borax, 13 g of sodium lauryl sulfate, 12 kg of water, and 0.2 g of copper (II) acetate were added, and these materials were heated with stirring. Once the temperature of the contents reached 70°C, introduction of 0.52 kg of oxygen was initiated. Stirring was continued such that the reaction temperature was maintained at 70°C, and the introduction of oxygen was terminated after 12 hours, followed by purging of the reaction system with nitrogen. Subsequently, after adjusting the pH of the resulting slurry to be 9.3 with an addition of 25% sulfuric acid, the temperature was gradually increased to remove water and unreacted 2,6-xylenol by distillation. Then, the temperature inside the reactor was cooled to 70°C, and the pH of the reaction solution was adjusted to be 6.5 with an addition of 25% sulfuric acid, after which 1.75 kg of water and 6.05 kg of isopropyl alcohol were added, and the resultant was stirred for 30 minutes with the temperature being maintained at 60°C. Thereafter, the temperature was maintained at 60°C, and the thus obtained slurry was subjected to a solid-liquid separation treatment using a centrifuge, and the resulting solid in the centrifuge was rinsed with 5 kg of 60°C hot water. The thus obtained solid was dried in a vacuum dryer, whereby a phenol mixture was obtained.

The formulations of the above-obtained phenol mixtures (ratios (% by weight) of PPE, TMBPL, ED, DPQ, and XNL) are shown in Tables 1 and 2.

**Table 1 [% by weight]**

| | Example | | | | |
|---|---|---|---|---|---|
| | 1 | 2 | 3 | 4 | 5 |
| PPE | 0.7 | 1.0 | 3.3 | 5.0 | 7.0 |
| TMBPL | 96.6 | 96.2 | 93.2 | 91.6 | 88.7 |
| ED | 0.0 | 0.1 | 0.1 | 0.1 | 0.2 |
| DPQ | 1.3 | 1.5 | 2.6 | 3.2 | 4.0 |
| XNL | 1.4 | 1.2 | 0.8 | 0.1 | 0.1 |

**Table 2 [% by weight]**

| | Comparative Example | | | | |
|---|---|---|---|---|---|
| | 1 | 2 | 3 | 4 | 5 |
| PPE | 0.3 | 0.1 | 0.1 | 0.1 | 10.0 |
| TMBPL | 99.0 | 99.8 | 93.6 | 86.8 | 85.3 |
| ED | 0.1 | 0.0 | 0.1 | 0.1 | 0.2 |
| DPQ | 0.1 | 0.0 | 2.7 | 2.7 | 4.4 |
| XNL | 0.5 | 0.1 | 3.5 | 10.3 | 0.1 |

### [Production of Epoxy Resins]

### [Example 6]

To a 2-L three-necked flask equipped with a thermometer, a stirrer, and a condenser, 121 g of the phenol mixture obtained in the above-described Example 1, 555 g of epichlorohydrin, and 200 g of isopropyl alcohol were added, and these materials were heated to 40°C, after which 91 g of a 48.5% aqueous sodium hydroxide solution was added dropwise thereto with stirring over a period of 1 hour. During this period, the temperature was gradually increased such that the temperature inside the system was 65°C at the completion of the dropwise addition. Subsequently, the temperature was maintained at 65°C for 30 minutes to allow a reaction to proceed. After the completion of the reaction, byproduct salt and excess sodium hydroxide were removed by washing with water. Then, excess epichlorohydrin and isopropyl alcohol were removed from the product under reduced pressure to obtain an epoxy resin. The thus obtained epoxy resin was dissolved in 250 g of methyl isobutyl ketone, and 2 g of a 48.5% aqueous sodium hydroxide solution was added to allow a reaction to proceed for 1 hour at a temperature of 65°C. After the completion of the reaction, sodium hydroxide was neutralized with an addition of sodium dihydrogen phosphate dihydrate, and byproduct salt was removed. Thereafter, insoluble matters were separated by filtration, and methyl isobutyl ketone was then completely removed under reduced pressure, whereby a target epoxy resin was obtained. The epoxy equivalent thereof is shown in Table 3.

### [Example 7]

An epoxy resin was synthesized in the same manner as in Example 6, except that the phenol mixture obtained in Example 2 was used. The epoxy equivalent of the epoxy resin is shown in Table 3.

### [Example 8]

An epoxy resin was synthesized in the same manner as in Example 6, except that the phenol mixture obtained in Example 3 was used. The epoxy equivalent of the epoxy resin is shown in Table 3.

### [Example 9]

An epoxy resin was synthesized in the same manner as in Example 6, except that the phenol mixture obtained in Example 4 was used. The epoxy equivalent of the epoxy resin is shown in Table 3.

### [Example 10]

An epoxy resin was synthesized in the same manner as in Example 6, except that the phenol mixture obtained in Example 5 was used. The epoxy equivalent of the epoxy resin is shown in Table 3.

### [Comparative Example 6]

An epoxy resin was synthesized in the same manner as in Example 6, except that the phenol mixture obtained in Comparative Example 1 was used. The epoxy equivalent of the epoxy resin is shown in Table 3.

### [Comparative Example 7]

An epoxy resin was synthesized in the same manner as in Example 6, except that the phenol mixture obtained in Comparative Example 2 was used. The epoxy equivalent of the epoxy resin is shown in Table 3.

### [Comparative Example 8]

An epoxy resin was synthesized in the same manner as in Example 6, except that the phenol mixture obtained in Comparative Example 3 was used. The epoxy equivalent of the epoxy resin is shown in Table 3.

### [Comparative Example 9]

An epoxy resin was synthesized in the same manner as in Example 6, except that the phenol mixture obtained in Comparative Example 4 was used. The epoxy equivalent of the epoxy resin is shown in Table 3.

### [Comparative Example 10]

An epoxy resin was synthesized in the same manner as in Example 6, except that the phenol mixture obtained in Comparative Example 5 was used. The epoxy equivalent of the epoxy resin is shown in Table 3.

**Table 3**

| | Example | | | | | Comparative Example | | | | |
|---|---|---|---|---|---|---|---|---|---|---|
| | 6 | 7 | 8 | 9 | 10 | 6 | 7 | 8 | 9 | 10 |
| Epoxy equivalent [g/eq] | 186 | 187 | 187 | 188 | 189 | 184 | 184 | 185 | 185 | 191 |

### [Production of Epoxy Resin Compositions and Cured Products: Examples 11 to 15 and Comparative Examples 11 to 15]

Epoxy resin compositions having the respective formulations shown in Tables 4 and 5 were each prepared using the epoxy resins obtained in Examples 6 to 10 and Comparative Examples 6 to 10. A phenol-aralkyl resin represented by Formula (2) (MEHC7800SS, manufactured by Meiwa Plastic Industries, Ltd., hydroxyl equivalent = 174 g/eq) was used as a phenolic curing agent, and triphenyl phosphine (HOKKO TPP, manufactured by Hokko Chemical Industry Co., Ltd.) was used as a curing accelerator. These materials were heated in an oven at 120°C for 2 hours and at 175°C for 6 hours to produce a cured product. The glass transition temperature (Tg) and the 5% weight reduction temperature of the thus obtained cured product were measured. The heat resistance was evaluated as "o" when the Tg was 130°C or higher and the 5% weight reduction temperature was 390°C or higher, while the heat resistance was evaluated as "×" when the Tg was 130°C or lower, or the 5% weight reduction temperature was 390°C or lower. In Tables below, "parts" represents "parts by weight".

### [Measurement of Glass Transition Temperature (Tg)]

The cured product was cut into a size of 5 cm in length, 1 cm in width, and 4 mm in thickness to obtain a test piece. Using a thermo-mechanical analyzer (DMS: EXSTAR 6100, manufactured by Seiko Instrument, Inc.) in a three-point bending mode, the test piece was heated at 1 Hz from 30°C to 250°C at a rate of 5°C/min, and the thus measured peak top temperature of loss tangent (tanδ) was defined as Tg.

### [Method of Measuring 5% Weight Reduction Temperature]

After shaving off 100 mg of the cured product, a portion of 10 mg was weighed therefrom and used as a sample. For this sample, a thermal analysis (heating rate: 5°C/min, measurement temperature range: 30°C to 350°C, air: flow rate = 200 mL/min) was performed using a thermal analyzer (TG/DTA: EXSTAR 7200, manufactured by Seiko Instrument, Inc.). The temperature at which the weight of the cured product was reduced by 5% was measured and defined as 5% weight reduction temperature.

**Table 4**

| | Example | | | | |
|---|---|---|---|---|---|
| | 11 | 12 | 13 | 14 | 15 |
| Example 6 [parts] | 100 | 0 | 0 | 0 | 0 |
| Example 7 [parts] | 0 | 100 | 0 | 0 | 0 |
| Example 8 [parts] | 0 | 0 | 100 | 0 | 0 |
| Example 9 [parts] | 0 | 0 | 0 | 100 | 0 |
| Example 10 [parts] | 0 | 0 | 0 | 0 | 100 |
| Phenolic curing agent [parts] | 94 | 94 | 93 | 93 | 92 |
| Curing accelerator [parts] | 1 | 1 | 1 | 1 | 1 |
| Tg [°C] | 130 | 135 | 137 | 134 | 130 |
| 5% weight reduction temperature [°C] | 390 | 393 | 395 | 397 | 400 |
| Heat resistance | ○ | ○ | ○ | ○ | ○ |

**Table 5**

| | Comparative Example | | | | |
|---|---|---|---|---|---|
| | 11 | 12 | 13 | 14 | 15 |
| Comparative Example 6 [parts] | 100 | 0 | 0 | 0 | 0 |
| Comparative Example 7 [parts] | 0 | 100 | 0 | 0 | 0 |
| Comparative Example 8 [parts] | 0 | 0 | 100 | 0 | 0 |
| Comparative Example 9 [parts] | 0 | 0 | 0 | 100 | 0 |
| Comparative Example 10 [parts] | 0 | 0 | 0 | 0 | 100 |
| Phenolic curing agent [parts] | 94 | 94 | 94 | 94 | 91 |
| Curing accelerator [parts] | 1 | 1 | 1 | 1 | 1 |
| Tg [°C] | 132 | 132 | 125 | 120 | 125 |
| 5% weight reduction temperature [°C] | 380 | 380 | 375 | 370 | 390 |
| Heat resistance | × | × | × | × | × |

### [Evaluation of Results]

From Tables 4 and 5, it is seen that the cured products of Examples 11 to 15 had superior heat resistance than the cured products of Comparative Examples 11 to 15.

## Claims

1. A phenol mixture, comprising 3,3',5,5'-tetramethyl-4,4'-biphenol as a main component,
wherein the phenol mixture further comprises more than 0.3% by weight but less than 10.0% by weight of a polyphenylene ether, and 1.3% by weight to 4.0% by weight of tetramethyldiphenoquinone.

2. An epoxy resin, obtained by reacting the phenol mixture according to claim 1 with an epihalohydrin.

3. An epoxy resin composition, comprising 0.01 to 1,000 parts by weight of a curing agent with respect to 100 parts by weight of the epoxy resin according to claim 1.

4. The epoxy resin composition according to claim 3, wherein the curing agent is at least one selected from the group consisting of a phenolic curing agent, an amine-based curing agent, a tertiary amine, an acid anhydride-based curing agent, an amide-based curing agent, and an imidazole.

5. The epoxy resin composition according to claim 3, further comprising an epoxy resin that is different from the epoxy resin contained in the epoxy resin composition.

6. A cured product, obtained by curing the epoxy resin composition according to claim 3.

7. An electric/electronic component, obtained by curing the epoxy resin composition according to claim 3.
